# EUROPÄISCHE PATENTSCHRIFT

(11) **EP 1 491 169 B1**
(45) Veröffentlichungstag und Bekanntmachung des Hinweises auf die Patenterteilung: **12.12.2007**
(21) Anmeldenummer: 04013552.7
(22) Anmeldetag: 09.06.2004
(51) Int. Cl.: A61F 5/055

(54) **Halskrawatte**
Cervical collar
Minerve

(30) Priorität: 24.06.2003 DE 20309801 U
(43) Veröffentlichungstag der Anmeldung: 29.12.2004
(73) Patentinhaber: Benckendorff, Petra, 22605 Hamburg (DE)
(72) Erfinder: Benckendorff, Petra, 22605 Hamburg (DE)
(74) Vertreter: Richter, Werdermann, Gerbaulet & Hofmann

(56) Entgegenhaltungen:
- DE-C- 4 495 250
- US-A- 1 964 962
- US-A- 3 320 950
- US-A- 3 696 810
- US-A1- 2002 052 568

## Beschreibung

Die Erfindung betrifft eine Halskrawatte gemäß dem Oberbegriff des Anspruches 1.

Zervikalstützen und so genannte Halskrawatten sind bekannt. Hierunter werden vorgefertigte Orthesen zur Entlastung der Halswirbelsäule und Stützung des Kopfes verstanden. Zervikalstützen werden insbesondere bei einem so genannten Schleudertrauma der Halswirbelsäule durch einen Verkehrsunfall verwendet. Bei einem Auffahrunfall wird der Kopf durch die einwirkenden Kräfte nach vorne und/oder hinten geschleudert, wobei das physiologische Bewegungsausmaß der Halswirbelsäule überschritten wird. Hierbei treten Zerrungen bis Zerreißungen der Weichteilstrukturen und knöcherne Impressionen auf, die zu einer Einschränkung der Halswirbelsäulenbeweglichkeit führen. Als Therapie wird zur Ruhigstellung häufig eine Zervikalstütze verwendet, die im Prinzip eine feste, ringförmige Halskrawatte mit Kinnauflage darstellt. Diese Halskrawatte ist so ausgeführt, dass sie die Nackenmuskulatur entlastet und den Kopf stützt. Der Nachteil der Zervikalstütze besteht darin, dass es bei längerem Tragen zu einer Schwächung der Schulter-Nacken-Muskulatur kommen kann, die schließlich dazu führen kann, dass die Patienten den Kopf nicht mehr eigenständig halten können.

Daher wird vielfach anstelle der nach dem Stand der Technik bekannten Zervikalstütze die so genannte Schanzsche Halskrawatte empfohlen. Diese besteht aus einer Watterolle in der Breite der Halshöhe, die gleichzeitig mit breiten Ideal- oder Cambrie-Binden so um den Hals gewickelt wird, dass die Binden die abrollende Watte ständig fixieren und komprimieren. Der Verband liegt breit auf den herabgezogenen Schultern und dem Brustbein auf und stützt den Kopf allseitig hoch. Gegebenenfalls wird die Halskrawatte durch eine übergewickelte, starke Binde verstärkt.

Da das zeitweise Abnehmen der Halskrawatte zur Vermeidung der Schwächung der Muskulatur erwünscht wird, sind fertige Halskrawatten vorgeschlagen worden, die wesentlich leichter sind und vom Patienten allein angelegt werden können. Diese Halskrawatten bestehen aus einer steifen Vlieseinlage mit einer Verstärkungsumhüllung sowie einer Schaumstoffumhüllung. Der Nachteil dieser Halskrawatten liegt darin, dass ihr Sitz nicht annähernd so gut und fest wie gewünscht ist.

Durch die US-A-4,819,622 ist eine Halskrawatte aus mehreren zu einem streifenförmigen Zuschnitt aneinander gefalteten Lagen und randseitig miteinander vernähten Lagen aus einem textilen Material, z. B. Frotteestoff, bekannt, deren Enden mit einem lösbaren Verschluss, insbesondere Klettverschluss, versehen sind. Da die aneinander gefalteten Lagen der Halskrawatte nur randseitig miteinander vernäht sind, ist bei dieser Halskrawatte keine ausreichende Eigenfestigkeit gegeben, um eine ausreichende stützende Wirkung zu erreichen, denn in den mittleren Abschnitten der Faltlagen ist keine ausreichende Festigkeit der Lagen zueinander gegeben. Hinzukommt noch, dass diese Halskrawatte aus einem textilen Material besteht, das darüberhinaus aufgrund seiner Gewebe- und Bindestruktur dehnbare Eigenschaften aufweist.

Es ist ferner durch die DE-A-44 95 250 eine Halskrawatte aus einem streifenförmigen Zuschnitt mit einem lösbaren Verschluss bekannt, die aus mehreren, engmaschig miteinander vernähten Lagen aus Molton besteht. Damit soll eine tag- und nachttragbare Halskrawatte bzw. Zervikalstütze mit hohem Tragekomfort geschaffen sein, die nicht nur zur Stützung bei Verletzungen und beim Halswirbelsyndrom einsetzbar ist, sondern auch von Reisenden, z. B. Flugpassagieren, auf langen Strecken zur Verbesserung der Kopfhaltung, insbesondere während der Ruhepausen und der Position während des Schlafens in Sitzposition bzw. in anderen Positionen verwendbar ist.

Die bekannten Halskrawatten sind endseitig mit Teilen eines Adhäsions- oder Klettverschlusses versehen und weisen relativ große Breiten auf, so dass sie sich im angelegten Zustand an dem Kinn-Seitenbereich und im seitlichen Nackenbereich abstützen, was zur Folge hat, dass diese Halskrawatten nicht beidseitig getragen werden können, was insbesondere auf die anatomische Formgebung zurückzuführen ist. Hinzukommt, dass der im Kehlkopfbereich liegende Verschlussbereich der Halskrawatten oftmals als störend empfunden wird, insbesondere dann, wenn Männer mit groß ausgebildeten Adamsäpfeln oder Personen, die aufgrund von Schilddrüsenerkrankungen Kehlkopfverdickungen oder kropfartige Vergrößerungen aufweisen, derartige Halskrawatten tragen, was oftmals zu Einengungen führt oder als Einengung empfunden wird.

Die US 2002/052568A1 offenbart eine Nackenstütze aus einem im angelegten Zustand der Nackenstütze in Längsrichtung der Wirbelsäule verlaufenden streifenförmigen Steg mit einer Anzahl von seitlichen, rippenartigen streifenförmigen Ausläufern, die um den Hals gelegt werden, wobei je zwei Ausläufer mit ihren sich gegenüberliegenden Enden vermittels an diesen vorgesehenen Verschlussteilen lösbar verbunden werden. Aufgrund dieser Ausgestaltung ist es nicht vermeidbar, dass es im angelegten Zustand die rippenartigen Ausläufer zu Einschnürungen im Halsbereich kommt, da diese Nackenstütze nicht vollflächig zur Anlage am Hals kommt. Das Entstehen von Druckstellen bleibt nicht aus.

Es ist daher Aufgabe der vorliegenden Erfindung, eine Halskrawatte der eingangs beschriebenen Art zu schaffen, die ein spannungsfreies Tragen, insbesondere im Kehlkopfbereich, ermöglicht und mit der die eingangs beschriebenen Nachteile vermieden werden, so dass im angelegten Zustand der Halskrawattenträger keine Einengung im Kehlkopfbereich und kein Beklemmungsgefühl verspürt.

Gelöst wird diese Aufgabe mit einer Halskrawatte mit den im Anspruch 1 angegebenen Merkmalen.

Erfindungsgemäß ist hiernach die Halskrawatte derart ausgebildet, dass die Halskrawatte aus einem längsverlaufenden, sich um den Hals im angelegten Zustand erstreckenden Zuschnitt besteht, der eine geradlinig verlaufende Längsseite und im Bereich seiner anderen Längsseite zwei nebeneinander liegende, im angelegten Zustand der Halskrawatte im seitlichen Nackenbereich unterhalb des Kopfes im seitlichen Nackenbereich anliegende, bogenförmige Erhöhungen aufweist, wobei die Länge der Erhöhungen etwa einem Zehntel der Gesamtlänge des Zuschnittes entspricht, dessen beide Enden unter Ausbildung von je zwei zungenartigen Endabschnitten mit einem zwischen den Endabschnitten liegenden in Zuschnittslängsrichtung verlaufenden Schlitz oder Einkerbung versehen ist, wobei die zungenartigen Endabschnitte Verschlussteile tragen.

Aufgrund dieser Ausgestaltung wird eine Halskrawatte mit einem hohen Tragekomfort geschaffen, der insbesondere darin liegt, dass die im Kehlkopfbereich liegenden Verschlussteile der angelegten Halskrawatte weder auf den Kehlkopf noch auf irgendwelche Kehlkopfverdickungen einen unangenehmen Druck ausüben. Das Gefühl einer Einengung wird vermieden; das Tragen der Halskrawatte stellt für den Träger keine Belastung dar; es treten keine Druckstellen auf und da die Verschlussteile im Kehlkopfbereich liegen, ist ein Anlegen und Abnehmen der Halskrawatte mühelos und durchaus auch von älteren Menschen durchführbar.

Weiterbildungen der Erfindung sind in den Unteransprüchen beschrieben.

Ausführungsbeispiele der Erfindung sind in den Zeichnungen dargestellt. Es zeigen:
- Fig. 1: eine Ansicht der Halskrawatte und
- Fig. 2: die Außenseite der Halskrawatte.

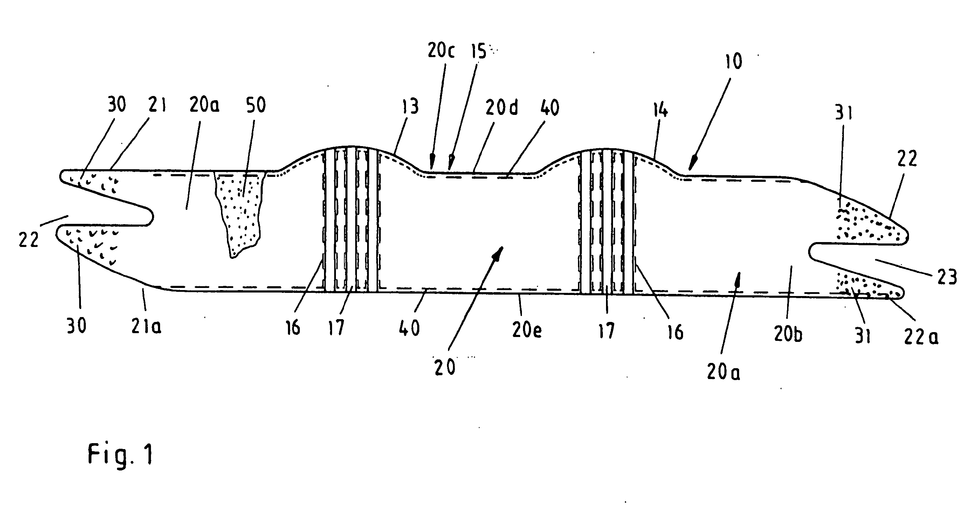

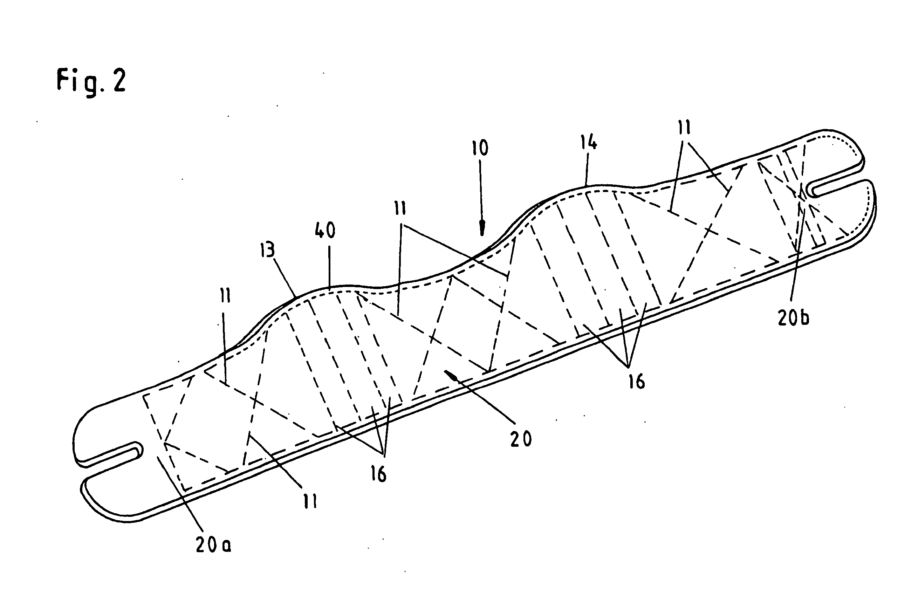

Die in Fig. 1 und 2 dargestellte Halskrawatte 10 besteht z. B. aus mehreren Lagen aus Molton, die engmaschig durch rautenweise Nähte 11 miteinander vernäht sind. Diese Nähte bzw. Abnäher erhöhen die Eigenfestigkeit des Grundmaterials Molton. Bei Molton handelt es sich um ein beidseitig gerauhtes, weiches, leinwandbindiges Baumwollgewebe. Jedoch auch andersartig ausgebildete Materialien können für die Herstellung der Halskrawatte verwendet werden.

Die Halskrawatte 10 besteht aus einem relativ schmalen Zuschnitt 20. Bevorzugterweise beträgt die Breite des Zuschnittes 20 ein Sechstel der Gesamtlänge des Zuschnittes.

An den Endseiten sind Klettverschlusselemente 30 und 31 vorgesehen, die ein leichtes Öffnen und Schließen der Halskrawatte ermöglichen. Die Halskrawatte besitzt eine Breite b, die etwa der Länge des zu stützenden Halsteiles entspricht. Die Länge der Halskrawatte, die im abgewickelten Zustand dargestellt ist, entspricht etwa dem Halsumfang. Die Halskrawatte besitzt zwei nebeneinanderliegende Erhöhungen 13 und 14, die beim Anlegen der Halskrawatte an den Halsseiten bzw. dem seitlichen Nackenbereich anliegen und hier stützen, während der Bereich 15 im Nacken zur Anlage kommt. In diesen erhöhten Bereichen und sich über die gesamte Breite erstreckend sind Taschen 16 mit glatten Innenwänden vorgesehen, in denen Versteifungsstäbchen 17 eingesteckt sind. Im Bereich der Taschen 16 sind keine Materialabnäher vorgesehen. In jedem Bereich kann eine Tasche 16 mit einem Versteifungsstäbchen 17 oder es können auch mehrere Taschen 16, z. B. drei Taschen 16, mit Verstärkungsstäbchen 17 vorgesehen sein. Im vorliegenden Fall befinden sich jeweils drei Taschen mit drei Versteifungsstäbchen in der Halskrawatte. An der Außenseite sind über den Taschen und den Versteifungsstäbchen liegende Lagen miteinander vernäht, so dass sich gerade in den Bereichen 13 und 14 optimale Verstärkungen ergeben. Das Molton-Material ist eingefärbt. Hier können die verschiedensten Farben zur Anwendung gelangen.

Der Zuschnitt 20 der Halskrawatte 10 weist im Bereich seiner Enden 20a, 20b als Verschluss die Teile 30, 31 eines Adhäsions- oder Klettverschlusses auf, wobei auch andersartig ausgestaltete Verschlüsse zum Einsatz gelangen können.

Jedes Ende 20a, 20b des Zuschnittes 20 ist unter Ausbildung von zwei zungenartigen Endabschnitten 21, 21 a und 22, 22a mit einem in Zuschnittslängsrichtung verlaufenden Schlitz oder Einkerbung 22, 23 versehen. Diese zungenartigen Endabschnitte 22, 22a tragen die Verschlussteile 30, 31.

Der Zuschnitt 20 der Halskrawatte 10 ist in seinem Randbereich 20c mit einer Polsterung 40 aus einem weichen Polstermaterial versehen, so dass im angelegten Zustand der Halskrawatte 10 keine Druckstellen im Halsbereich auftreten können. -

Die beiden Erhöhungen 13, 14 sind an einer der beiden Längsseiten 20d, 20e des Zuschnittes 20 ausgebildet; sie stellen im seitlichen Nackenbereich anliegende bogenförmige Abschnitte von geringer Höhe dar. Die im Randbereich 20c des Zuschnittes 20 ausgebildete Polsterung 40 erstreckt sich auch über den Randbereich der beiden Erhöhungen 13, 14.

Die Länge einer jeden Erhöhung 13, 14 entspricht in etwa einem Zehntel der Gesamtlänge des Zuschnittes 20.

Mindestens eine Seite des Zuschnittes 20 der Halskrawatte 10 ist mit einer hyperaemisierenden oder wärmehaltenden Auflage 50 versehen. Da die Halskrawatte 10 aufgrund ihrer Ausgestaltung beidseitig tragbar ist, besteht die Möglichkeit, die Seitenflächen des Zuschnittes 10 mit unterschiedlichen Farbgebungen oder Stoffarten zu versehen.

Die Versteifungsstäbe 17 können auch als stabförmige Pelotten ausgebildet sein, die aus einem auf die jeweilige gewünschte Härte eingestellten Kunststoff bestehen, z. B. aus Silicon-Kautschuk oder einem anderen geeigneten Kunststoff, so dass bei angelegter Halskrawatte vermittels der derart ausgebildeten Versteifungsstäbe eine massierende Wirkung erreicht wird.

## Patentansprüche

1. Halskrawatte aus einem streifenförmigen Zuschnitt, die sich im angelegten Zustand der Halskrawatte um den Hals herum erstreckt und deren Enden Verschlussteile tragen, wobei
die Halskrawatte (10) aus einem längsverlaufenden, sich um den Hals im angelegten Zustand erstreckenden Zuschnitt (20) besteht, der eine geradlinig verlaufende Längsseite (20e) und im Bereich seiner anderen Längsseite (20d) zwei nebeneinander liegende, im angelegten Zustand der Halskrawatte im seitlichen Nackenbereich unterhalb des Kopfes im seitlichen Nackenbereich anliegende, bogenförmige Erhöhungen (13, 14) aufweist, **dadurch gekennzeichnet, daß** die Länge der Erhöhungen etwa einem Zehntel der Gesamtlänge des Zuschnittes (20) entspricht, dessen beide Enden (20a, 20b) unter Ausbildung von je zwei zungenartigen Endabschnitten (21, 21 a; 22, 22a) mit einem zwischen den Endabschnitten liegenden in Zuschnittslängsrichtung verlaufenden Schlitz oder Einkerbung (22; 23) versehen ist, wobei die zungenartigen Endabschnitte Verschlussteile (30, 31) tragen, so daß die im Kehlkopfbereich liegenden Verschlussteile der angelegten Halskrawatte weder auf den Kehlkopfbereich noch auf irgendwelche Kehlkopfverdickungen einen unangenehmen Druck ausüben.

2. Halskrawatte nach Anspruch 1,
**dadurch gekennzeichnet,**
**dass** der Zuschnitt (20) der Halskrawatte (10) in seinem Randbereich (20c) mit einer Polsterung (40) aus einem weichen Polstermaterial versehen ist.

3. Halskrawatte nach einem der Ansprüche 1 oder 2,
**dadurch gekennzeichnet,**
**dass** mindestens eine Seite des Zuschnittes (20) der Halskrawatte (10), mit einer hyperaemisierenden oder wärmehaltenden Auflage (50) versehen ist.

## Claims

1. Cervical collar made of a strip-shaped blank which extends around the neck when it is in the laid state and the ends of which support closure parts, whereby the cervical collar (10) is made of a longitudinal blank (20) which extends around the neck, when it is in the laid state, which has a linear longitudinal side (20e) and in the area of its other longitudinal side (20d) two arc-shaped raised parts (13, 14) situated the one besides the other, bearing in the laid state of the cervical collar, in the lateral neck area under the head in the lateral neck area,
**characterized in**
**that** the length of the raised parts approximately corresponds to one tenth of the overall length of the blank (20), both ends (20a, 20b) of which constitute respectively two tongue-type end sections (21, 21 a; 22, 22a) with a slit or notch (22, 23) situated between the end sections in the blank longitudinal direction, whereby the tongue-type end sections support closure parts (30, 31) so that the closure parts of the laid cervical collar which are situated in the larynx area do not exert an unpleasant pressure neither on the larynx area, nor on any larynx swelling.

2. Cervical collar according to claim 1,
**characterized in**
**that** the blank (20) of the cervical collar (10) is provided in its edge area (20c) with a padding (40) made of a soft pad material.

3. Cervical collar according to any of the claims 1 or 2,
**characterized in**
**that** at least one side of the blank (20) of the cervical collar (10) is provided with a hyperaemic function improving or heat retaining coating (50).

## Revendications

1. Minerve constituée par une découpe en forme de bande qui s'étend, lorsque la minerve est à l'état posé, autour du cou et dont les extrémités portent des pièces de fermeture, la minerve (10) étant constituée par une découpe (20) dans le sens de la longueur qui s'étend autour du cou, à l'état posé, qui présente un côté longitudinal en ligne droite (20e) et, dans la zone de son autre côé longitudinal (20d), deux bosses arquées (13, 14) situées l'une à côté de l'autre, qui adhèrent, lorsque la minerve est à l'état posé, dans la zone latérale du cou au-dessous de la tête dans la zone latérale de la nuque,
**caractérisée en ce**
**que** la longueur des bosses correspond approximativement à un dizième de la longueur totale de la découpe (20) dont les deux extrémités (20a, 20b), en constituant respectivement deux sections d'extrémité de type languette (21, 21a ; 22, 22a), sont pourvues d'une fente ou encoche (22, 23) située entre les sections d'extrémité dans le sens longitudinal de la découpe, les sections d'extrémité de type languette portant des pièces de fermeture (30, 31) si bien que les pièces de fermeture situées dans la zone du larynx de la minerve posée n'exercent de pression désagréable ni sur la zone du larynx, ni sur quelque gonflement du larynx.

2. Minerve selon la revendication 1,
**caractérisée en ce**
**que** la découpe (20) de la minerve (10) est pourvue, dans sa zone marginale (20c), d'un rembourrage (40) en un matériau de rembourrage souple.

3. Minerve selon l'une des revendications 1 ou 2,
**caractérisée en ce**
**qu'**au moins un côté de la découpe (20) de la minerve (10) est pourvu d'un appui favorisant l'hyperémie ou assurant la rétention de chaleur (50).
